# EUROPEAN PATENT APPLICATION

(11) **EP 4 811 656 A1**
(43) Date of publication of application: **23.09.2026**
(21) Application number: 26161939.9
(22) Date of filing: 03.03.2026
(51) Int. Cl.: H03F 1/34, H03F 3/45, H03F 3/00

(54) **SWITCHED CAPACITOR DUAL-PATH DC CANCELLATION FRONT-END AMPLIFIER**

(30) Priority: 21.03.2025 US 202519086954
(71) Applicant: Avago Technologies International Sales Pte. Limited, Singapore 768923 (SG)
(72) Inventor: Lu, Junjie, Irvine, CA, 92620 (US); Guo, Jing, Irvine, CA, 92618 (US); Jiang, Xicheng, Irvine, CA, 92612 (US)
(74) Representative: Grünecker Patent- und Rechtsanwälte PartG mbB

(57) **Abstract**

A device may include a signal input, an operational amplifier (op-amp) comprising a noninverting op-amp input coupled to the signal input, an inverting op-amp input, and an op-amp output, a capacitor comprising a plurality of ports and a switched capacitor arrangement. The switched capacitor arrangement includes a first switch coupled between a first capacitor port and the inverting op-amp input, a second switch coupled between a second capacitor port and the op-amp output, a third switch coupled between a third capacitor port and the signal input, and a fourth switch coupled between a fourth capacitor port and ground. The device may process electrophysiology signals by implementing switched-capacitor dual-path DC cancellation to achieve offset and low-frequency interference rejection while maintaining high input impedance and signal amplification.

## Description

### BACKGROUND

Electrophysiology signals provide valuable insights into various physiological processes and are widely used in wearable devices, medical diagnostics and health monitoring. These signals, which include electrocardiograms (ECG), electromyograms (EMG), and electroencephalograms (EEG), typically have small amplitudes in the range of microvolts to millivolts. Acquiring and processing these weak signals presents challenges, particularly in the presence of larger DC offsets and low-frequency interference that can arise from electrode-skin interfaces and environmental factors.

Wearable devices for continuous health monitoring have gained popularity due to their potential for early detection of health issues and personalized healthcare. However, the use of dry electrodes in these devices, while more comfortable for long-term wear, introduces additional complexities in signal acquisition. Dry electrodes often have higher impedance and are more susceptible to motion artifacts compared to traditional wet electrodes used in clinical settings.

### BRIEF DESCRIPTION OF FIGURES

Non-limiting and non-exhaustive examples are described with reference to the following figures.
FIGS. 1A-1B illustrates a circuit diagram of an example front-end amplifier in various switching states, in accordance with one or more embodiments of the disclosure.
FIGS. 1C-1D illustrates a circuit diagram of an example front-end amplifier in various switching states, in accordance with one or more embodiments of the disclosure.
FIG. 2 illustrates a state transition diagram showing the operation of a switched capacitor circuit, in accordance with one or more embodiments of the disclosure.
FIG. 3 illustrates a block diagram of a wearable device incorporating a front-end amplifier, in accordance with one or more embodiments of the disclosure.
FIG. 4 illustrates a manufacturing process for producing a front-end amplifier, in accordance with one or more embodiments of the disclosure.

### DETAILED DESCRIPTION

The following description sets forth exemplary aspects of the present disclosure. It should be recognized, however, that such description is not intended as a limitation on the scope of the present disclosure. Rather, the description also encompasses combinations and modifications to those exemplary aspects described herein.

The terminology used herein may be for describing various examples only and does not limit the disclosure. Unless otherwise defined, all terms, including technical and scientific terms, used herein may have the same meaning as commonly understood by one of ordinary skill in the art to which this disclosure pertains and after an understanding of the disclosure of this application. Terms, such as those defined in commonly used dictionaries, may be interpreted as having a meaning that is consistent with their meaning in the context of the relevant art and the disclosure of this application. Although the present technology has been described by referring to certain examples, workers skilled in the art may recognize that changes may be made in form and detail without departing from the scope of the discussion.

Conventional front-end amplifiers for electrophysiology signals may struggle to effectively process weak signals in the presence of larger DC offsets and low-frequency interference. These systems may have difficulty maintaining signal integrity, particularly when used with dry electrodes in wearable devices. The challenges may be compounded by the higher impedance and increased susceptibility to motion artifacts associated with dry electrodes, which may lead to degraded signal quality and reduced accuracy in health monitoring applications.

The described technology may address these challenges by providing a front-end amplifier system that incorporates a dual-path DC cancellation network. The dual-path approach may allow for rejection of DC offsets and low-frequency interference while maintaining the desired signal components.

This technology may improve the performance of wearable health monitoring devices by enabling acquisition and processing of weak electrophysiology signals. The dual-path DC cancellation may enhance the signal-to-noise ratio by attenuating unwanted low-frequency components while amplifying the desired signal frequencies. Additionally, the system may be suited for use with electrodes, potentially improving long-term monitoring capabilities and user comfort in wearable applications.

The described technology may be implemented in various wearable devices and systems that utilize electrodes for electrophysiology (EP) signal acquisition. These may include smart watches, fitness trackers, and health monitoring bands that may continuously track heart rate, detect arrhythmias, or monitor stress levels. The technology may also be incorporated into smart clothing, such as shirts or chest straps with integrated electrodes, which may provide ECG monitoring during physical activities.

In addition to wearable devices, the technology may be implemented in portable medical devices used for ambulatory monitoring, such as Holter monitors or event recorders. These devices may benefit from improved signal quality and reduced motion artifacts, potentially leading to diagnoses and patient outcomes. The technology may also find applications in consumer electronics like smartphones or tablets, which may incorporate electrodes in their casings or accessories for on-demand ECG measurements. Smart home devices, such as bathroom scales or sleep monitoring systems, may utilize this technology to provide health data to users. In clinical settings, the technology may be integrated into patient monitoring systems, including bedside monitors and telemetry devices, to improve the quality of continuous EP signal acquisition. The technology may also be used in specialized medical equipment such as EEG headsets for brain activity monitoring or EMG systems for muscle activity assessment. Furthermore, the technology may be applied in non-medical fields where biosignal monitoring may be relevant, such as in sports performance analysis systems, fatigue monitoring devices for drivers or pilots, or even in human-computer interaction devices that use muscle activity as input signals.

While the front-end amplifiers described below may be discussed with respect to signal acquisition from a dry electrode of a wearable device, the front-end amplifiers may be applied to any suitable system where weak signal amplification and DC offset rejection may be desired. The principles and architecture of these amplifiers may be adapted for use in various fields beyond wearable health monitoring. For example, in atomic force microscopy (AFM), where extremely small deflections of a cantilever may need to be detected, a front-end amplifier with high input impedance, low noise, and effective DC cancellation may support measuring the tiny electrical signals generated by the AFM probe as the AFM probe scans a sample surface. In this application, the amplifier's ability to reject low-frequency drift and environmental interference while amplifying the desired high-frequency components of the signal may enhance the resolution and stability of the AFM measurements.

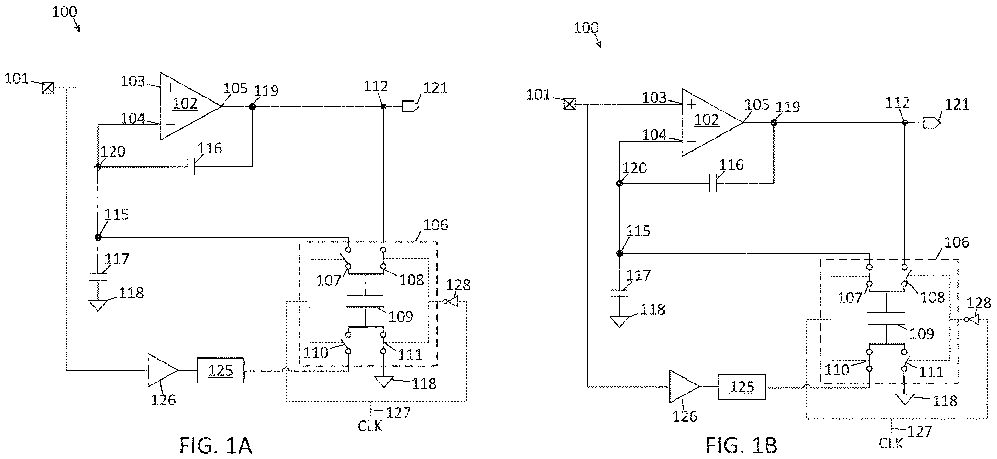

FIGS. 1A and 1B illustrate a front-end amplifier 100 for processing electrophysiology (EP) signals. Front-end amplifier 100 may include a signal input 101, an operational amplifier (op-amp) 102, a switched capacitor 106, and an antialiasing filter 125. In some cases, front-end amplifier 100 may be implemented using discrete components on a printed circuit board. For example, op-amp 102 may be a discrete op-amp chip, while switched capacitor 106 and antialiasing filter 125 may be constructed using discrete resistors and capacitors. Alternatively, front-end amplifier 100 may be implemented as an integrated circuit, with components fabricated on a single semiconductor die.

Front-end amplifier 100 may include a signal input 101 connected to an op-amp 102. Op-amp 102 may comprise a non-inverting input 103, an inverting input 104, and an op-amp output 105. Op-amp 102 may be a high-gain operational amplifier designed to process weak electrophysiology signals, utilizing a folded-cascode architecture to provide high gain and wide bandwidth while maintaining stability. It may include multiple stages such as a differential input stage, gain stage, and output buffer stage, and may be implemented using CMOS technology. The non-inverting input 103 may be connected to signal input 101, which may comprise a CMOS transistor. This input transistor may be a thick-oxide IO MOS transistor to reduce input leakage current, potentially below 10 pA, resulting in an effective input impedance of more than 100 GΩ. The gate of this CMOS transistor may be isolated from the rest of the circuitry by the oxide insulator, resulting in an extremely high input impedance. This configuration may help prevent attenuation of the input electrophysiology signal, particularly when used with low-conductance dry electrodes. The inverting input 104 may receive the feedback signal, while op-amp output 105 may provide the amplified and processed signal. Op-amp 102 may incorporate chopper stabilization techniques to reduce low-frequency noise and offset. The physical implementation of op-amp 102 may vary depending on specific application requirements, being fabricated as part of an integrated circuit using standard CMOS processes or implemented using discrete components on a printed circuit board for prototyping or specialized applications.

Front-end amplifier 100 may include a feedback capacitor network comprising second capacitor 116 and third capacitor 117. Second capacitor 116 may be connected between op-amp output 105 and inverting input 104 via feedback capacitor connections 119 and 120. Third capacitor 117 may be connected to ground 118. The capacitances of second capacitor 116 and third capacitor 117 may establish the signal gain of front-end amplifier 100. Front-end amplifier 100 may be configured to amplify weak electrophysiology (EP) signals by a factor of 2x-100x, which may help reduce noise and power requirements for subsequent signal processing stages. The ratio of these capacitances may be adjusted to set the amplification factor according to the transfer function T(s)=VOUT(s)/VIN(s)= (C1+Cfb)/Cfb, where C1 represents the capacitance of third capacitor 117 and Cfb represents the capacitance of second capacitor 116.

This capacitive feedback approach may offer several advantages: capacitors do not generate thermal noise, potentially improving SNR; no DC current flows through capacitors, potentially reducing power consumption; the capacitor network may facilitate implementation of a high-pass transfer function; and capacitors may have better matching and lower temperature drift than resistors in modern CMOS processes, potentially improving gain accuracy and temperature stability.

Front-end amplifier 100 may incorporate both feedback and feedforward signal paths to process the input signal. This dual-path configuration may enable effective rejection of DC offsets and low-frequency interference while preserving desired signal components. For example, dual path DC cancellation may be implemented through switched capacitor 106, which combines both the input (feedforward) and output (feedback) signals before sending them to the inverting input 104 of op-amp 102. As DC offset and low-frequency interference may be substantially larger than the signal of interest, thorough DC cancellation can be beneficial, and even small DC leakage in conventional approaches may be undesirable for some applications.

Switched capacitor 106 may combine signals from both the feedback and feedforward paths. The feedback path may include feedback connection 112 from op-amp output 105. The feedforward path may connect signal input 101 to switched capacitor 106 through antialiasing filter 125. During operation, switched capacitor 106 alternately samples the feedback signal from op-amp output 105 and the feedforward signal from antialiasing filter 125. The combined signal incorporating components from both paths is then provided to inverting input 104 via connection 115 for further processing by op-amp 102.

Switched capacitor 106 may be included in front-end amplifier 100. Switched capacitor 106 may comprise a first capacitor 109 comprising a plurality of ports including a first switch 107, a second switch 108, a first capacitor 109, a third switch 110, and a fourth switch 111. For instance, these switches may be implemented as CMOS transmission gates, each consisting of parallel NMOS and PMOS transistors. For example, first capacitor 109 may be formed using metal-insulator-metal (MIM) capacitor structures. The switches and capacitor may be configured to implement switching operations between different circuit nodes. A feedback connection 112 may connect to the output path of op-amp 102. The circuit may include a connection 115 coupled to second capacitor 116 and third capacitor 117, with ground connections 118. Second capacitor 116 and third capacitor 117 may also be implemented as MIM capacitors. In some cases, the CMOS implementation of switched capacitor 106 may allow for low power consumption and high integration density.

Front-end amplifier 100 may include a front-end amplifier output 121 that provides the processed signal output. Antialiasing filter 125 may be positioned between an input buffer 126 and switched capacitor 106. Input buffer 126 may be a unity gain amplifier that maintains the high input impedance of front-end amplifier 100.

The front-end amplifier 100 may operate in two distinct phases (ϕ1 and ϕ2), as illustrated in FIGS. 1A and 1B. These operational phases may be controlled by a clock source 127 and an off-phase clock source 128, as shown in FIGS. 1A and 1B. The clock source 127 may be coupled to the first switch 107 and the third switch 110 to control their operation, while the off-phase clock source 128 may be coupled to the second switch 108 and the fourth switch 111 to control their operation. Here, the clock signals from clock source 127 and off-phase clock source 128 are 180 degrees out of phase with respect to each other. During each phase, one pair of switches is closed while the other pair is open. The switching between these phases may enable the front-end amplifier 100 to implement both summing and low-pass filter functions through the switched capacitor 106 arrangement. The term "off-phase" is used here for purposes of explanation to distinguish between and describe the phase relationship the two clock sources. It should be understood that the clock signals could be switched without varying the operation of the circuit. For example, clock source 127 could control switches 108 and 111 while off-phase clock source 128 controls switches 107 and 110.

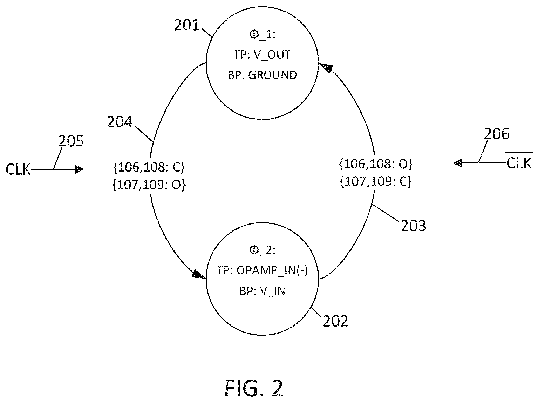

FIG. 2 illustrates a state transition diagram showing the operation of the switched capacitor 106 illustrated in FIGS. 1A and 1B. The diagram depicts a first state 201 and a second state 202, representing different configurations of the switched capacitor 106 circuit, where TP indicates the charge state of the top capacitor plate, and BP indicates the charge state of the bottom plate. Here, the terms "top plate" and "bottom plate" refer to the illustration of the switched capacitor 106 and do not indicate any particular physical implementation or configuration. A first transition 204 connects from the first state 201 to the second state 202, while a second transition 203 connects from the second state 202 back to the first state 201. A clock signal 205 and an off-phase clock signal 206 may control these transitions. In some cases, the clock signal 205 may be generated by the clock source 127, while the off-phase clock signal 206 may be generated by the off-phase clock source 128.

In the first state 201, as shown in FIG. 1A, the second switch 108 and the fourth switch 111 may be closed, while the first switch 107 and the third switch 110 may be open. This configuration may be controlled by the clock signal 205 from the clock source 127. In this configuration, the first capacitor 109 may be connected between the feedback connection 112 and the ground 118. During this phase, the first capacitor 109 may sample the output voltage from the front-end amplifier output 121.

In the second state 202, as shown in FIG. 1B, the first switch 107 and the third switch 110 may be closed, while the second switch 108 and the fourth switch 111 may be open. This configuration may be controlled by the off-phase clock signal 206 from the off-phase clock source 128. In this configuration, the first capacitor 109 may be connected between the output of the antialiasing filter 125 and the inverting input 104 of the op-amp 102. During this phase, the voltage stored on the first capacitor 109 from the first phase, along with the input signal from the antialiasing filter 125, may be transferred to the inverting input 104 via the connection 115.

The switching between these two states may implement a dual-path DC cancellation network. In the first state 201, the first capacitor 109 may sample the output signal (feedback path), and in the second state 202, the first capacitor 109 may add the input signal (feedforward path) to the previously sampled output signal. This combined signal may then be applied to the inverting input 104 of the op-amp 102. The clock signal 205 and the off-phase clock signal 206 may control the timing of these operations, ensuring proper synchronization of the dual-path DC cancellation network.

The switched capacitor 106, in conjunction with the second capacitor 116, may form a low-pass filter in the feedback path. For signals with frequencies much lower than the switching frequency of the clock signal 205 and the off-phase clock signal 206, the switched capacitor 106 may behave as an equivalent resistor. This equivalent resistor, together with the second capacitor 116, may create a low-pass filter. The low-pass filter in the feedback path may result in a high-pass characteristic for the overall transfer function from the signal input 101 to the front-end amplifier output 121. The term "low-pass filter" may apply to a circuit that allows signals below a certain frequency to pass through while attenuating signals above that frequency. The frequency at which the filter begins to attenuate higher frequency signals is known as the corner frequency. In particular, examples are discussed with respect to the -3dB corner frequency, which is the frequency at which higher frequencies are attenuated by at least 3dB (e.g., the gain is -3dB). In particular, the term 'low' does not imply any particular corner frequency, an example low-pass filter might be configured with a corner frequency from Hz to GHz (or higher).

The signal transfer function (STF) of the front-end amplifier 100 may be characterized by its frequency response, which may be influenced by various system parameters. In some aspects, the STF may be represented in the Laplace domain as: $STF \left(s\right) = \frac{OUT \left(s\right)}{IN \left(s\right)} = \frac{s \left({C}_{1}+{C}_{fb}\right)}{{fC}_{sc} + {sC}_{fb}}$ where s is the complex frequency variable, C₁ represents third capacitor 117, C_{fb} represents second capacitor 116, f is the switching frequency of clock signal 205 and off-phase clock signal 206, and C_{sc} is the value of first capacitor 109 in the switched capacitor 106. This transfer function may exhibit high-pass characteristics, attenuating DC and low-frequency components while passing higher frequency signals. As the frequency increases, the magnitude of the STF may approach a constant gain determined by the ratio of capacitors (C₁ + C_{fb}) / C_{fb}. The high-pass corner frequency of the front-end amplifier 100 may be adjustable by modifying the switching frequency of the clock signal 205 and off-phase clock signal 206. Increasing the switching frequency may shift the corner frequency higher, while decreasing it may lower the corner frequency. This flexibility may allow the system to be tuned for different electrophysiology signal types or to adapt to varying noise conditions. In some implementations, the high-pass corner frequency may be proportional to the switching frequency and the value of first capacitor 109, and inversely proportional to the value of second capacitor 116. By adjusting the clock frequency, the high-pass characteristics may be modified without changing any hardware components. Additionally, the system response may be further tailored by selecting appropriate values for the third capacitor 117, second capacitor 116, and first capacitor 109. These electrical parameters may influence the gain, bandwidth, and noise performance of the front-end amplifier 100. The combination of frequency and component value adjustments may provide a versatile platform for optimizing the front-end amplifier's performance across various applications and operating conditions, as illustrated by the two-state operation shown in FIG. 2.

In some cases, the high-pass corner frequency of front-end amplifier 100 may be determined by the switching frequency of clock signal 205 and off-phase clock signal 206. The high-pass corner frequency may be proportional to the switching frequency, the value of first capacitor 109, and inversely proportional to the value of second capacitor 116. By modifying the switching frequency, the high-pass corner frequency may be adjusted without altering any hardware components. The transfer function may approach 0 magnitude at DC or low-frequency (as s approaches 0), which may verify the high-pass characteristics of the overall system. The -3 dB corner frequency of the high-pass function may be given by: ${f}_{hpf} = \frac{f ∗ {C}_{sc}}{2 π ∗ {C}_{fb}}$

The corner frequency of the switched capacitor 106 low-pass filter may depend on the ratio of capacitors (Csc/Cfb) and the switching frequency (f). In some cases, the capacitor ratio may be controlled by lithographic precision and may achieve up to 0.1% accuracy without trimming. The switching frequency may be governed by an external crystal oscillator clock. In some implementations, the switched capacitor 106 low-pass filter may not require calibration to achieve accuracy. In some modern CMOS processes, capacitors and switches may have low process variation, potentially less than 1%. This low variability may contribute to the accuracy and consistency of the switched capacitor 106 low-pass filter. The switched capacitor 106 low-pass filter may exhibit temperature stability characteristics. In contrast, a conventional RC implementations may determine the high-pass filter corner frequency by the product of RC values and modern CMOS processes may have up to ±20% process variation for resistors.

Further, as described above, the switched capacitor 106 low-pass filter function may be tunable. In some cases, the low-pass filter corner frequency may need to be varied, for example, to accommodate electrophysiology signals in different frequency ranges or to attenuate specific interference bands. As the low-pass filter corner may be proportional to the switching frequency f, the corner frequency may be changed by modifying the switching frequency. At electrophysiology signal frequencies (f_{sig}>>f_{hpf}), the signal transfer function may be simplified to (C₁+C_{fb})/C_{fb}. At these frequencies, front-end amplifier 100 may have a constant gain determined by the capacitor ratio.

In some cases, the antialiasing filter 125 may be included between the input buffer 126 and the switched capacitor 106 to prevent aliasing of high-frequency noise components. The antialiasing filter 125 may be a simple RC filter with a corner frequency higher than the signal bandwidth but lower than half the switching frequency.

The non-inverting input 103 of the op-amp 102 may remain connected to the signal input 101 throughout both operational phases, maintaining the high input impedance characteristic of the front-end amplifier 100. This configuration may help prevent attenuation of weak electrophysiology signals when using low-conductance dry electrodes.

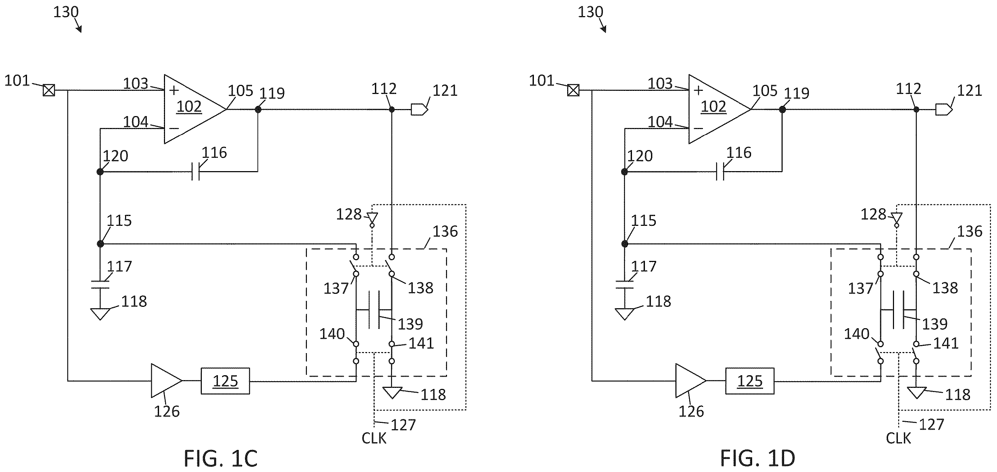

FIGS. 1C and 1D illustrate another example front end amplifier 130. Compared to front end amplifier 100, front end amplifier 130 includes another example switched capacitor 136. In this example, the switched capacitor 136 includes a first capacitor 139 comprising a plurality of ports. A first capacitor port is connected to the non-inverting op-amp input 104 via a first switch 137, a second capacitor port is connected to the op-amp output 112 via a second switch 138, a third capacitor port is connected to the signal input 101 via a third switch 140, and a fourth capacitor port is connected to ground 118 via a fourth switch 141. Compared to the capacitor 109, the first switch 137 and the third switch 140 are connected in parallel to a first terminal of capacitor 139 (e.g., the TP of capacitor 139), while the second switch 138 and the fourth switch 141 are connected in a parallel to a second terminal of capacitor 139 (e.g., the BP of capacitor 139). Similarly, the clocking arrangement differs from switched capacitor 106. Here, the first switch 137 and second switch 138 are connected to a first common clock source 128, while the third switch 140 and the fourth switch 141 are connected to a second common clock source 127.

In this example, in a first phase (FIG. 1C), switches 140 and 141 are closed while switches 137 and 138 are open. In this phase, the first capacitor 139 samples the input signal via the path from signal input 101 to ground 118, storing the input voltage. In a second phase (FIG. 1D), the switches 137 and 138 are closed while the switches 140 and 141 are open. In this phase, the capacitor 139 samples the output signal and the combined voltage is transferred to the inverting op-amp input 104 (e.g., parallel to the second capacitor 116). The switched capacitor 136 implements the summing and low pass filtering as described above. In further examples, other switched capacitor configurations may be implemented, such as other connectivity or switching behavior. Any suitable configuration may be implemented without departing from the scope of the described technology.

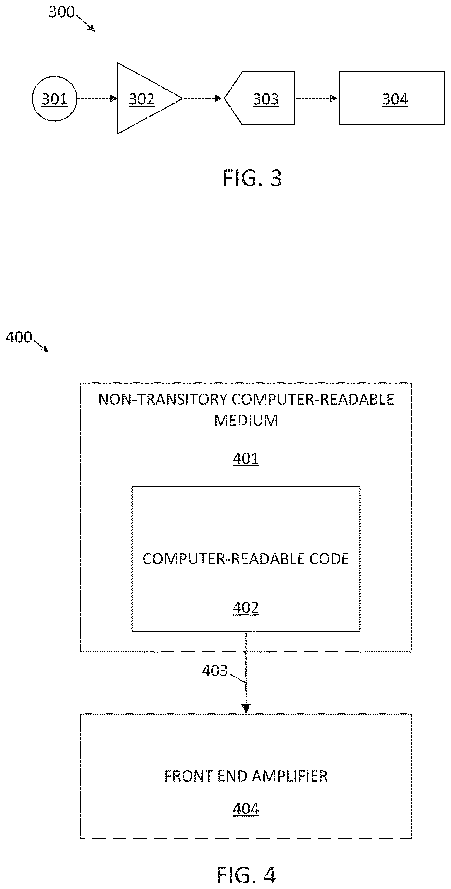

FIG. 3 illustrates a block diagram of a wearable device 300. Wearable device 300 may include an electrode 301, a front-end amplifier 302, an analog-to-digital converter (ADC) 303, and a processor 304. Wearable device 300 may be implemented in various form factors such as smartwatches, fitness bands, chest straps, or smart clothing with integrated sensors. The device may incorporate additional components not explicitly shown, such as input/output interfaces (e.g. displays, buttons, haptic feedback), wireless communication modules (e.g. Bluetooth, Wi-Fi), memory storage, and power management systems. Other sensors may also be included, such as accelerometers, gyroscopes, or optical heart rate sensors, to provide complementary physiological and motion data.

The illustrated components may be implemented in various manners. For example, front-end amplifier 302, ADC 303, and processor 304 may be integrated as components of a system-on-chip (SOC), such as a very-large-scale integration (VLSI) integrated circuit. This high level of integration may allow for a compact and power-efficient design, potentially reducing the overall size and power consumption of wearable device 300. As another example, front-end amplifier 302 may be integrated directly with electrode 301 to form a single sensor unit, while ADC 303 and processor 304 may be integrated into a separate application-specific integrated circuit (ASIC). In other cases, wearable device 300 may be implemented using discrete components. For instance, the components may be mounted on one or more printed circuit boards (PCB).

Electrode 301 may be configured to interface with a user's skin and collect electrophysiology signals. In some implementations, electrode 301 may be designed for extended use and biocompatibility, making electrode 301 suitable for continuous health monitoring applications. For example, electrode 301 may comprise a conductive polymer material or a metal-coated fabric that provides electrical contact with the skin.

Front-end amplifier 302 may be coupled to electrode 301 and may receive the collected electrophysiology signals as input. Front-end amplifier 302 may be an implementation of front-end amplifier 100, as described in relation to FIG. 1. Front-end amplifier 302 may be configured to amplify and condition the weak electrophysiology signals collected by electrode 301.

In some cases, front-end amplifier 302 may include a signal input coupled to electrode 301, an op-amp with a non-inverting input connected to the signal input, an inverting input, and an op-amp output. Front-end amplifier 302 may also include a switched capacitor arrangement with a first switch coupled between a first capacitor port and the inverting op-amp input, a second switch coupled between the first capacitor port and the op-amp output, a third switch coupled between a second capacitor port and the signal input, and a fourth switch coupled between the second capacitor port and ground. This switched capacitor arrangement may implement both summing and low-pass filter functions through its switching operations.

ADC 303 may be coupled to the output of front-end amplifier 302. ADC 303 may be configured to convert the amplified and conditioned analog signals from front-end amplifier 302 into digital format. This conversion may allow for easier processing and analysis of the electrophysiology signals by subsequent digital components. For example, ADC 303 may be a 24-bit ADC with a sampling rate of 1 kHz, which may be suitable for capturing the frequency content of many electrophysiology signals.

Processor 304 may be connected to the output of ADC 303. Processor 304 may be configured to receive and process the digitized electrophysiology signals. In some cases, processor 304 may perform various signal processing tasks, such as filtering, feature extraction, or pattern recognition, to derive information from the electrophysiology signals. Processor 304 may include, for example, a digital signal processor, a microcontroller, or a hybrid/combined processor incorporating features of both.

The signal flow in wearable device 300 may begin with electrode 301 collecting electrophysiology signals from the user's skin. These signals may then be passed to front-end amplifier 302, which may amplify the weak signals and reject unwanted DC offset and low-frequency interference. The amplified and conditioned analog signals may then be converted to digital format by ADC 303. Finally, the digitized signals may be processed by processor 304, for example, to extract physiological information, to transmit to another system, etc.

This configuration of wearable device 300 may allow for efficient and accurate processing of electrophysiology signals in a compact, wearable form factor. The use of electrode 301 may enable long-term, comfortable monitoring, while front-end amplifier 302 may ensure high-quality signal acquisition even with the challenges posed by electrodes, such as high impedance and potential motion artifacts.

In some implementations, front-end amplifier 302 may include tuning capabilities to adjust its performance characteristics. The tuning may be achieved by modifying the switching frequency of the switched capacitor arrangement within front-end amplifier 302. By changing the switching frequency, the high-pass corner frequency of front-end amplifier 302 may be adjusted without altering any hardware components. This tuning capability may allow wearable device 300 to adapt to different types of electrophysiology signals or varying noise conditions in different environments. In some cases, the tuning may be performed automatically by processor 304 or other circuitry based on detected signal characteristics.

FIG. 4 illustrates a manufacturing process 400 for producing a front-end amplifier, such as front-end amplifier 302 described in relation to FIG. 3. Manufacturing process 400 may include a non-transitory computer-readable medium 401, computer-readable code 402, and a semiconductor design and fabrication process 403.

Non-transitory computer-readable medium 401 may be any type of storage device capable of storing computer-readable instructions. In some cases, non-transitory computer-readable medium 401 may be a hard drive, solid-state drive, flash memory, or other form of persistent storage. Non-transitory computer-readable medium 401 may contain computer-readable code 402, which may include instructions for implementing the design of front-end amplifier 404. Computer-readable code 402 can be stored in any non-transitory computer-readable medium 401 such as a semiconductor medium, solid-state medium, magnetic medium, optical medium, etc. For example, computer-readable medium 401 might comprise a memory device, storage device, registers (e.g., static RAM), cache device, etc.

Computer-readable code 402 may comprise a set of instructions that, when executed by a computer system, may define the structure and functionality of front-end amplifier 404. These instructions may include specifications for the various components of front-end amplifier 404, such as op-amp 102 and the switched capacitor arrangement. In some cases, computer-readable code 402 may also include parameters for the capacitor values and switching frequencies used in the front-end amplifier design. In some configurations, the computer-readable code 402 is used at one or more stages of a semiconductor design and fabrication process 403, including an electronic design automation (EDA) stage, to fabricate 403 an integrated circuit 404. The computer-readable code 402 may additionally or alternatively enable the definition, modelling, simulation, verification and/or testing of an apparatus embodying the concepts described herein.

For example, the computer-readable code 402 can be embodied in code defining a hardware description language (HDL) representation of the circuitry 404. For example, the code may define a register-transfer-level (RTL) abstraction of one or more logic circuits for defining an apparatus embodying the concepts. The code may define an HDL representation of the one or more logic circuits embodying the apparatus in Verilog, SystemVerilog, Chisel, or VHDL (Very High-Speed Integrated Circuit Hardware Description Language) as well as intermediate representations such as FIRRTL. Computer-readable code 402 may provide definitions embodying the circuitry using system-level modelling languages such as SystemC and SystemVerilog or other behavioral representations of the concepts that can be interpreted by a computer to enable simulation, functional and/or formal verification, and testing of the circuitry.

In some examples, the computer-readable code 402 may define a low-level description of integrated circuit components that embody concepts described herein, such as one or more netlists or integrated circuit layout definitions, including representations such as GDSII. The one or more netlists or other computer-readable representation of integrated circuit components may be generated by applying one or more logic synthesis processes to an RTL representation to generate definitions for use in fabrication 403 of an apparatus embodying the invention. In some examples, the one or more logic synthesis processes can generate from the computer-readable code 402 a bitstream to be loaded into a field programmable gate array (FPGA) to configure the FPGA to implement the described circuitry 404. The FPGA may be deployed for the purposes of verification and test of the concepts prior to fabrication 403 in an integrated circuit or the FPGA may be deployed in a product directly.

Semiconductor design and fabrication process 403 may utilize computer-readable code 402 to produce front-end amplifier 404. This process may involve multiple stages of design, simulation, verification, and physical fabrication. In some cases, semiconductor design and fabrication process 403 may comprise any stage of a VLSI (Very Large Scale Integration) design and fabrication process.

The initial stages of semiconductor design and fabrication process 403 may involve translating the instructions in computer-readable code 402 into a detailed circuit design. This may include creating schematic diagrams and layout designs for front-end amplifier 404. During this stage, the specific connections and arrangements of components such as op-amp 102 and switched capacitor arrangement 106 may be defined.

Following the design phase, semiconductor design and fabrication process 403 may include simulation and verification steps. These steps may help ensure that front-end amplifier 404 meets the desired performance specifications, such as input impedance, gain, and frequency response characteristics. Simulations may be performed to verify the functionality of the dual-path DC cancellation network and the high-pass transfer function of front-end amplifier 404.

Once the design has been verified, semiconductor design and fabrication process 403 may proceed to the physical fabrication of front-end amplifier 404. This may involve a series of steps including wafer processing, photolithography, etching, and deposition of various materials to create the transistors, resistors, and capacitors that make up front-end amplifier 404.

After fabrication, front-end amplifier 404 may undergo testing and characterization to ensure compliance with the design specifications. This may include measurements of input impedance, gain accuracy, frequency response, and noise performance. The testing process may also verify the effectiveness of the DC offset and low-frequency interference rejection capabilities of front-end amplifier 404.

Manufacturing process 400 may enable the production of front-end amplifier 404 with consistent performance characteristics across multiple units. By utilizing non-transitory computer-readable medium 401 and computer-readable code 402, the design of front-end amplifier 404 may be easily modified and optimized for different applications or performance requirements.

Those skilled in the art will also appreciate the arrangement or interconnection of components such as "coupled," "connected," "on," "under," or similar wording allows for indirect connections, or intervening components or layers.

As used herein, unless otherwise limited or defined, "or" indicates a non-exclusive list of components or operations that may be present in any variety of combinations, rather than an exclusive list of components that may be present as alternatives to each other. For example, a list of "A, B, or C" indicates options of: A; B; C; A and B; A and C; B and C; and A, B, and C.

The term "or" is intended to indicate exclusive alternatives when preceded by terms of exclusivity, such as "either," "one of," or "exactly one of." A list preceded by "one or more" and including "or" to separate listed elements indicates options of one or more of any or all of the listed elements. For example, the phrases "one or more of A, B, or C" and "at least one of A, B, or C" indicate options of: one or more A; one or more B; one or more C; one or more A and one or more B; one or more B and one or more C; one or more A and one or more C; and one or more of each of A, B, and C. Similarly, a list preceded by "a plurality of" and including "or" to separate listed elements indicates options of multiple instances of any or all of the listed elements. For example, the phrases "a plurality of A, B, or C" and "two or more of A, B, or C" indicate options of: A and B; B and C; A and C; and A, B, and C.

The articles "a," "an," and "the" are intended to include the plural forms as well, unless the context clearly indicates otherwise.

Any mark, if referenced herein, may be common law or registered trademarks of third parties affiliated or unaffiliated with the applicant or the assignee. Use of these marks is by way of example and shall not be construed as descriptive or to limit the scope of disclosed or claimed embodiments to material associated with such marks.

The terms "comprises," "includes," and "has" specify the presence of stated features, numbers, operations, members, elements, and/or combinations thereof, but do not preclude the presence or addition of one or more other features, numbers, operations, members, elements, and/or combinations thereof.

Throughout the application, unless context indicates otherwise, ordinal numbers (e.g., first, second, third, etc.) may be used as an adjective for an element (i.e., any noun in the application). Although terms such as "first," "second," and "third" may be used herein to describe various members, components, regions, layers, or sections, these members, components, regions, layers, or sections are not to be limited by these terms. Rather, these terms are used to distinguish one member, component, region, layer, or section from another member, component, region, layer, or section. The use of ordinal numbers is not to imply or create any particular ordering of the elements nor to limit any element to being a single element unless expressly disclosed, such as by the use of the terms "before," "after," "single," and other such terminology. Rather, the use of ordinal numbers is to distinguish between the elements. By way of an example, a first element is distinct from a second element, and the first element may encompass more than one element and succeed (or precede) the second element in an ordering of elements. Thus, a first member, component, region, layer, or section referred to in examples described herein may also be referred to as a second member, component, region, layer, or section without departing from the teachings of the examples.

Certain operations of methods according to the technology, or of systems executing those methods, may be represented schematically in the figures or otherwise discussed herein. Unless otherwise specified or limited, representation in the figures of particular operations in particular spatial order may not require those operations to be executed in a particular sequence corresponding to the particular spatial order. Correspondingly, certain operations represented in the figures, or otherwise disclosed herein, may be executed in different orders than are expressly illustrated or described, as appropriate for particular examples of the technology. Further, in some examples, certain operations may be executed in parallel or partially in parallel, including by dedicated parallel processing devices, or separate computing devices configured to interoperate as part of a large system.
The following are further embodiments of the present invention:
1. A device, comprising:
   a signal input;
   an operational amplifier (op-amp) comprising a non-inverting op-amp input coupled to the signal input, an inverting op-amp input, and an op-amp output;
   a capacitor comprising a plurality of ports;
   a first switch coupled between a first capacitor port of the plurality of ports and the inverting op-amp input;
   a second switch coupled between a second capacitor port of the plurality of ports and the op-amp output;
   a third switch coupled between a third capacitor port of the plurality of ports and the signal input; and
   a fourth switch coupled between a fourth capacitor port of the plurality of ports and ground.
2. The device of embodiment 1, further comprising:
   a first clock source to control switching operations of the first switch and the third switch; and
   a second clock source to control switching operations of the second switch and the fourth switch.
3. The device of embodiment 1, further comprising:
   a first clock source to control switching operations of the first switch and the second switch; and
   a second clock source to control switching operations of the third switch and the fourth switch.
4. The device of embodiment 1, wherein:
   during a first operational phase, the second switch and the fourth switch are closed while the first switch and the third switch are open, and the capacitor is charged based on the op-amp output; and
   during a second operational phase, the first switch and the third switch are closed while the second switch and the fourth switch are open, and the capacitor is charged by the signal input, summing voltages from the first and second operational phases and performing a low-pass filter function.
5. The device of embodiment 1, wherein:
   during a first operational phase, the third switch and the fourth switch are closed while the first switch and the second switch are open, and the capacitor is charged based on the signal input; and
   during a second operational phase, the first switch and the second switch are closed while the third switch and the fourth switch are open, and the capacitor is charged by the op-amp output, summing voltages from the first and second operational phases and performing a low-pass filter function.
6. The device of embodiment 1, further comprising:
   an antialiasing filter coupled in series between the second capacitor port and the signal input.
7. The device of embodiment 6, further comprising:
   an input buffer coupled in series between the antialiasing filter and the signal input.
8. The device of embodiment 1, further comprising:
   a second capacitor coupled between the op-amp output and the inverting op-amp input; and
   a third capacitor coupled between ground and the inverting op-amp input;
   wherein the first capacitor is coupled to the inverting op-amp input in parallel to the second capacitor.
9. A system, comprising:
   an electrode; and
   a front-end amplifier comprising:
      a signal input coupled to the electrode;
      an operational amplifier (op-amp) comprising a non-inverting op-amp input coupled to the signal input, an inverting op-amp input, and an op-amp output;
      a capacitor comprising a plurality of ports;
      a first switch coupled between a first capacitor port of the plurality of ports and the inverting op-amp input;
      a second switch coupled between a second capacitor port of the plurality of ports and the op-amp output;
      a third switch coupled between a third capacitor port of the plurality of ports and the signal input; and
      a fourth switch coupled between a fourth capacitor port of the plurality of ports and ground.
10. The system of embodiment 9, wherein the front-end amplifier further comprises:
   a first clock source to control switching operations of the first switch and the third switch;
   a second clock source to control switching operations of the second switch and the fourth switch.
11. The system of embodiment 9, wherein the front-end amplifier further comprises:
   a first clock source to control switching operations of the first switch and the second switch; and
   a second clock source to control switching operations of the third switch and the fourth switch.
12. The system of embodiment 9, wherein:
   during a first operational phase, the second switch and the fourth switch are closed while the first switch and the third switch are open, and the capacitor is charged based on the op-amp output; and
   during a second operational phase, the first switch and the third switch are closed while the second switch and the fourth switch are open, and the capacitor is charged by the signal input, summing voltages from the first and second operational phases and performing a low-pass filter function.
13. The system of embodiment 9, wherein:
   during a first operational phase, the third switch and the fourth switch are closed while the first switch and the second switch are open, and the capacitor is charged based on the signal input; and
   during a second operational phase, the first switch and the second switch are closed while the third switch and the fourth switch are open, and the capacitor is charged by the op-amp output, summing voltages from the first and second operational phases and performing a low-pass filter function.
14. The system of embodiment 9, wherein the front-end amplifier further comprises:
   an antialiasing filter coupled in series between the second capacitor port and the signal input; and
   an input buffer coupled in series between the antialiasing filter and the signal input.
15. The system of embodiment 9, wherein the front-end amplifier further comprises:
   a second capacitor coupled between the op-amp output and the inverting op-amp input; and
   a third capacitor coupled between ground and the inverting op-amp input;
   wherein the first capacitor is coupled to the inverting op-amp input in parallel to the second capacitor.
16. The system of embodiment 9, further comprising an analog-to-digital converter (ADC) coupled to the op-amp output.
17. A non-transitory computer-readable medium comprising stored instructions to manufacture a device, the device comprising:
   a signal input;
   an operational amplifier (op-amp) comprising a non-inverting op-amp input coupled to the signal input, an inverting op-amp input, and an op-amp output;
   a capacitor comprising a plurality of ports;
   a first switch coupled between a first capacitor port of the plurality of ports and the inverting op-amp input;
   a second switch coupled between a second capacitor port of the plurality of ports and the op-amp output;
   a third switch coupled between a third capacitor port of the plurality of ports and the signal input; and
   a fourth switch coupled between a fourth capacitor port of the plurality of ports and ground.
18. The non-transitory computer-readable medium of embodiment 17, wherein the device further comprises:
   a first clock source to control switching operations of the first switch and the third switch;
   a second clock source to control switching operations of the second switch and the fourth switch.
19. The non-transitory computer-readable medium of embodiment 17, wherein the device further comprises:
   a first clock source to control switching operations of the first switch and the second switch; and
   a second clock source to control switching operations of the third switch and the fourth switch.
20. The non-transitory computer-readable medium of embodiment 17, wherein the device further comprises:
   an antialiasing filter coupled in series between the second capacitor port and the signal input; and
   an input buffer coupled in series between the antialiasing filter and the signal input.

## Claims

1. A device, comprising:
a signal input;
an operational amplifier (op-amp) comprising a non-inverting op-amp input coupled to the signal input, an inverting op-amp input, and an op-amp output;
a capacitor comprising a plurality of ports;
a first switch coupled between a first capacitor port of the plurality of ports and the inverting op-amp input;
a second switch coupled between a second capacitor port of the plurality of ports and the op-amp output;
a third switch coupled between a third capacitor port of the plurality of ports and the signal input; and
a fourth switch coupled between a fourth capacitor port of the plurality of ports and ground.

2. The device of claim 1, further comprising:
a first clock source to control switching operations of the first switch and the third switch; and
a second clock source to control switching operations of the second switch and the fourth switch.

3. The device of claim 1 or claim 2, further comprising:
a first clock source to control switching operations of the first switch and the second switch; and
a second clock source to control switching operations of the third switch and the fourth switch.

4. The device of any of the preceding claims, wherein:
during a first operational phase, the second switch and the fourth switch are closed while the first switch and the third switch are open, and the capacitor is charged based on the op-amp output; and
during a second operational phase, the first switch and the third switch are closed while the second switch and the fourth switch are open, and the capacitor is charged by the signal input, summing voltages from the first and second operational phases and performing a low-pass filter function.

5. The device of any of the preceding claims, wherein:
during a first operational phase, the third switch and the fourth switch are closed while the first switch and the second switch are open, and the capacitor is charged based on the signal input; and
during a second operational phase, the first switch and the second switch are closed while the third switch and the fourth switch are open, and the capacitor is charged by the op-amp output, summing voltages from the first and second operational phases and performing a low-pass filter function.

6. The device of any of the preceding claims, further comprising:
an antialiasing filter coupled in series between the second capacitor port and the signal input,
in particular wherein the device further comprises an input buffer coupled in series between the antialiasing filter and the signal input.

7. The device of any of the preceding claims, further comprising:
a second capacitor coupled between the op-amp output and the inverting op-amp input; and
a third capacitor coupled between ground and the inverting op-amp input;
wherein the first capacitor is coupled to the inverting op-amp input in parallel to the second capacitor.

8. A system, comprising:
an electrode; and
a front-end amplifier comprising:
a signal input coupled to the electrode;
an operational amplifier (op-amp) comprising a non-inverting op-amp input coupled to the signal input, an inverting op-amp input, and an op-amp output;
a capacitor comprising a plurality of ports;
a first switch coupled between a first capacitor port of the plurality of ports and the inverting op-amp input;
a second switch coupled between a second capacitor port of the plurality of ports and the op-amp output;
a third switch coupled between a third capacitor port of the plurality of ports and the signal input; and
a fourth switch coupled between a fourth capacitor port of the plurality of ports and ground.

9. The system of claim 8, wherein the front-end amplifier further comprises:
a first clock source to control switching operations of the first switch and the third switch;
a second clock source to control switching operations of the second switch and the fourth switch.

10. The system of claim 8 or claim 9, wherein the front-end amplifier further comprises:
a first clock source to control switching operations of the first switch and the second switch; and
a second clock source to control switching operations of the third switch and the fourth switch.

11. The system of any of claims 8 to 10, wherein:
during a first operational phase, the second switch and the fourth switch are closed while the first switch and the third switch are open, and the capacitor is charged based on the op-amp output; and
during a second operational phase, the first switch and the third switch are closed while the second switch and the fourth switch are open, and the capacitor is charged by the signal input, summing voltages from the first and second operational phases and performing a low-pass filter function.

12. The system of any of claims 8 to 11, wherein:
during a first operational phase, the third switch and the fourth switch are closed while the first switch and the second switch are open, and the capacitor is charged based on the signal input; and
during a second operational phase, the first switch and the second switch are closed while the third switch and the fourth switch are open, and the capacitor is charged by the op-amp output, summing voltages from the first and second operational phases and performing a low-pass filter function.

13. The system of any of claims 8 to 12, wherein the front-end amplifier further comprises:
an antialiasing filter coupled in series between the second capacitor port and the signal input; and
an input buffer coupled in series between the antialiasing filter and the signal input.

14. The system of any of claims 8 to 13,
wherein the front-end amplifier further comprises:
a second capacitor coupled between the op-amp output and the inverting op-amp input; and
a third capacitor coupled between ground and the inverting op-amp input;
wherein the first capacitor is coupled to the inverting op-amp input in parallel to the second capacitor;
and/or
wherein the system further comprises an analog-to-digital converter (ADC) coupled to the op-amp output.

15. A non-transitory computer-readable medium comprising stored instructions to manufacture a device according to any of claims 1 to 7.
